# NEW EUROPEAN PATENT SPECIFICATION

(11) **EP 0 494 941 B2**
(45) Date of publication and mention of the opposition decision: **24.07.2002**
(45) Mention of the grant of the patent: 21.06.1995
(21) Application number: 90915220.9
(22) Date of filing: 02.10.1990
(51) Int. Cl.: A61F 13/56, A41H 43/04

(54) **A METHOD OF SECURING AN ELASTIC BAND**
VERFAHREN ZUR BEFESTIGUNG EINES ELASTISCHEN STREIFENS
PROCEDE DE FIXATION D'UN ELASTIQUE

(30) Priority: 04.10.1989 SE 8903256
(43) Date of publication of application: 22.07.1992
(73) Proprietor: SCA Hygiene Products AB, 405 03 Göteborg (SE)
(72) Inventor: KLING, Robert, S-511 63 Skene (SE); WALL, Berth-Ove, S-483 00 Landvetter (SE)
(74) Representative: Harrison, Michael Charles
(86) International application number: SE9000631
(87) International publication number: WO9104724

(56) References cited:
- EP-A- 0 023 804
- EP-A- 0 119 827
- EP-A- 0 217 032
- EP-A- 0 219 969
- EP-A- 0 274 752
- EP-B- 0 112 055
- US-A- 4 157 719
- US-A- 4 726 976

## Description

The present invention relates to a method of securing an elastic band between two layers of material which are made at least partially of meltable material. The invention also relates to an elastic band secured between two layers of material and intended for use in articles intended for one-time use only, such as disposable diapers, sanitary napkins, surgical dressings, protective clothing or the like, and also to a diaper which includes such an elastic band.

In the manufacture of articles intended for one-time use only, such as disposable diapers or the like, it is desirable that these articles can be worn comfortably, will function effectively and fit properly, besides having an attractive appearance. These attributes are often compared with the attributes of corresponding multi-use articles. Such articles are traditionally manufactured from very soft, woven or knitted textile materials which are comfortable to wear and in which great care has been taken in cutting and sewing the materials from which the articles are made.

When manufacturing disposable articles, however, it is not possible to use expensive materials or to apply complicated and expensive manufacturing methods, since the articles would then demand an unreasonably high price. Instead, the traditional textile materials are usually replaced with plastic film, different types of non-woven fabric and tissue layers. Techniques such as gluing and welding are used, instead of sewing the articles together.

One particular problem in this respect is that of providing elasticated edges, for instance elasticated leg, waist and sleeve bands. Normally, elastication in the form of elastic tape, bands or threads is secured along the edges of the article to be elasticated with the aid of melt adhesive. This method has several drawbacks, however. For instance, the glue has an uncontrollable stiffening effect on the elastic devices used, therewith impairing the elasticity of said devices. In the case of disposable diapers, for instance, the elastic devices, or elastication, is normally placed between two casing layers, normally a plastic film and a non-woven fabric layer. The elastic devices are mounted between the casing layers and fastened thereto while in a stretched state, whereafter the elastic devices return to their non-stretched, shorter state and gather together those parts of the casing layers to which the devices are attached. As a result, there is formed, primarily in the plastic film relatively sharp folds which are liable to chafe the skin of the wearer. Gluing of the elastic devices also causes the elasticated edge to become hard and chafing. These known elasticated edges are thus not as comfortable as would be desired.

Another drawback is that the formation of folds in the outer layers, or casing layers, results in the formation of channels between the elastic devices and the wearer's skin. This is particularly disadvantagous when the elastic devices are placed around the leg and waist parts of diapers, since these channels provide routes through which excreted body fluids can escape from the diaper.

Neither can the known elastications be considered to satisfy the desire for an attractive, fabriclike appearance, but instead result in a wrinkled and shapeless finished article.

Difficulties are also experienced in the manufacture of articles where the elastication is glued thereto. The main problem is that the glue, which may be a melt glue or a thermosetting glue for instance, requires a certain amount of time for heating and cooling or drying the glue after it has been applied. During this time period, it is necessary to maintain the elastication in a stretched state, which presents a problem in a continuous manufacturing process. Furthermore, the necessity of handling a glue requires the provision of expensive special-duty equipment for avoiding troublesome occurrences, such as the adhesion of glue to machine rolls and knives.

Hitherto, it has not been found possible to provide in a simple and ready fashion elasticated edges which possess varying degrees of elasticity within different parts of the edges, or where certain parts of said edges are completely inelastic. Various methods of providing elastication in limited areas, or with varying degrees of pretensioning, in a continuous manufacturing process have been proposed, for instance, in GB-A-2 113 983, EP-A-184 072, US-A-4 642 151, US-A-4 425 173, US-A-4 486 192, US-A-4 081 301, US-A-4 711 683 and US-A-4 642 109.

EP-A1-23 804 discloses an elastic member disposed between first and second substrates of flexible gatherable material, said elastic member including a plurality of interconnected elastic elements defining apertures therebetween, and the first and second substrates being adhesively secured together through at least some of said apertures. The connection between the substrates through the pre-formed apertures in the elastic member is accomplished by the intermediate of an adhesive applied to one of the substrates or to the elastic member. The adhesive is pressed into the apertures with the aid of compression rollers or the like. In order for the adhesive to effectively secure the substrates to each other, one of the compression rollers should be made of resilient material. Such a method of securing is solely applicable for adhesive connections.

SE-B-436 393 discloses a diaper in which a strip of plastic material is welded in between an outer sheet and an inner sheet. There are no perforations through which the inner and outer sheets are bonded to each other. Both the inner sheet and the outer sheet are bonded to the strips by welding thereto and not to each other.

The present invention now provides a simple method of securing an elastic band between two material layers while avoiding the drawbacks experienced with earlier known elastication.

In accordance with the invention, this is accomplished in that perforations in the form of holes and/or slots are formed in the elastic band; in that the band is placed between the two material layers; in that the material layers opposite said perforations are mutually bonded by heat fusion through said perforations; and in that the perforations are formed in the elastic band in the same operation as the material layers are fused together.

The invention also relates to an elastic band secured between two material layers.

When such an elastic band is fastened onto an article which is intended for one-time use only, there is obtained a soft, non-chafing elastic edge. This is mainly due to the fact that no glue is used to fasten the elastication, but that the elastication is held mechanically between two mutually bonded material layers. Consequently, both the elastication and the material layers will retain their original softness and suppleness. This is also due to the fact that the elastic band can move to a certain extent in relation to the material layers surrounding said band, thereby enabling said layers to pleat or fold independently of one another when the elastic band relaxes. Distinct from the earlier known elastications, which gather the surrounding material layers into sharp, chafing folds, the material layers will form around an inventive elastic band soft, rounded folds which bulge outwardly from the elastication. This is because the material layers are bonded to the elastic band by mutually spaced bonds, those parts of the material layers located between the bonds are not bonded at all.

It should be mentioned that in the present context, the expression elastic band refers to an elongated elastic body whose width has a much greater extension than the perforations formed therein in accordance with the invention.

A further advantage provided by an inventive elastic band resides in the possibility of achieving varying degrees of stretchability or elasticity within different regions of one and the same elastic band, simply by changing the bonding pattern. This feature is particularly useful in the case of all-in-one type diapers, for instance. Diapers of this kind comprise an absorbent pad which is enclosed between a liquid permeable, inner casing layer and a liquid-impermeable, outer casing layer or backing sheet. The elastication is normally applied along the edges of the absorbent pad, so as to form elasticated waist and leg edges. The diapers also include self-adhesive fastener tabs, which are attached to the corners of the diaper on the backing sheet, i.e. on that part of the diaper which is distal from the wearer in use. The tabs function to hold together the sides of the diaper, so as to obtain a trouser-like configuration around the abdomen of the wearer. The fastener tabs are secured over the front part of the diaper, i.e. that part of the diaper which faces towards the wearer in use.

When securing the fastener tabs, it is desirable that the surfaces of the diaper material are as smooth as possible. This applies particularly to that part of the front part of the diaper on which the fastener tabs are intended to be secured in use. If this part of the diaper is wrinkled and irregular, the fastener tabs will not grip securely and there is a risk that the diaper will not be held together during use. An advantage is also afforded when the tab attachments on the rear part of the diaper have inelastic, smooth regions, since this facilitates securing of the tabs to the diaper during manufacture, and also provides more positive attachment of the fastener tabs. If the tape-fastening areas are elastic and the fastener tabs are secured to the diaper with the elastic devices are broken down in a stretched state, there is a serious risk that the tabs will loosen from the diaper when the elastic devices are relaxed from their stretched state and subsequently contract. The present invention, however, enables totally inelastic regions to be readily provided, by selecting an elastic-device bonding pattern such that the elastic devices are broken down in these regions and loose their elasticity.

It is also possible to adjust the degree of elasticity around the leg openings of the diaper, for instance. The greatest elasticity is preferably found within those regions in which the elastic devices are attached to the crotch region of the diaper, i.e. that part of the diaper which, in use, is intended to lie between the wearer's thighs. On the other hand, there is preferably chosen in those parts of the elastic devices which extend along the front and rear portion of the diaper a bonding pattern which will reduce the elasticity of the elastic devices.

A further advantage afforded by an elastic band configured in accordance with the invention is that it imparts an aesthetically attractive, well-tailored appearance to the finished article. The elastic band appears soft and comfortable, whereby the finished article looks to be extremely comfortable, to the naked eye.

When an inventive elastic band is secured to the surrounding material layers at solely mutually spaced bonding locations, which permits the material layers to fold or pleat freely in relation to the elastic device, there is obtained an airy elastic edge and the soft material folds give a certain padding effect. The risk of the skin becoming irritated is very small, since the folds in the material layers bonded to the elastic band provide a certain ventilating effect, which will keep the skin beneath the elastic band substantially dry during use. The elastic band may also be made from an open-cell foam material, so as to provide air ventilation through the actual band itself, provided that at least one of the surrounding material layers is permeable to air.

A number of advantages are also obtained when manufacturing an elastic band in accordance with the invention. For instance, the troublesome handling of glue is avoided, as is also the need of drying or heating and cooling melt adhesive. Furthermore, there is avoided the need of complicated devices for varied stretching of the elastic devices, or of devices for handling pieces clipped from said elastic devices.

The invention will now be described in more detail with reference to exemplifying embodiments thereof illustrated in the accompanying drawings.

Figure 1 illustrates a diaper according to a first embodiment of the invention, seen from the side which faces the wearer in use.

Figure 2 illustrates a diaper according to a second embodiment, seen from the side which faces the wearer in use.

Figure 3 is a sectional view taken on the line III-III on the diaper of Figure 2.

Figures 4-8 illustrates different methods of applying an inventive elastic device to the edge of an absorbent article.

Figure 9 illustrates schematically a process for the manufacture of an inventive elastic device.

Figures 10 and 11 illustrate different bonding patterns.

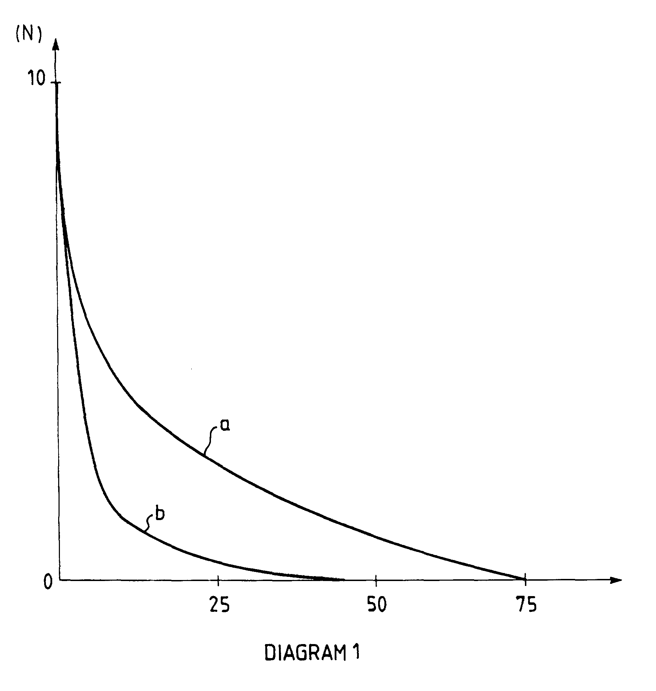

Figure 12 is a diagram which illustrates the contraction of two elastic bands having mutually different bonding patterns.

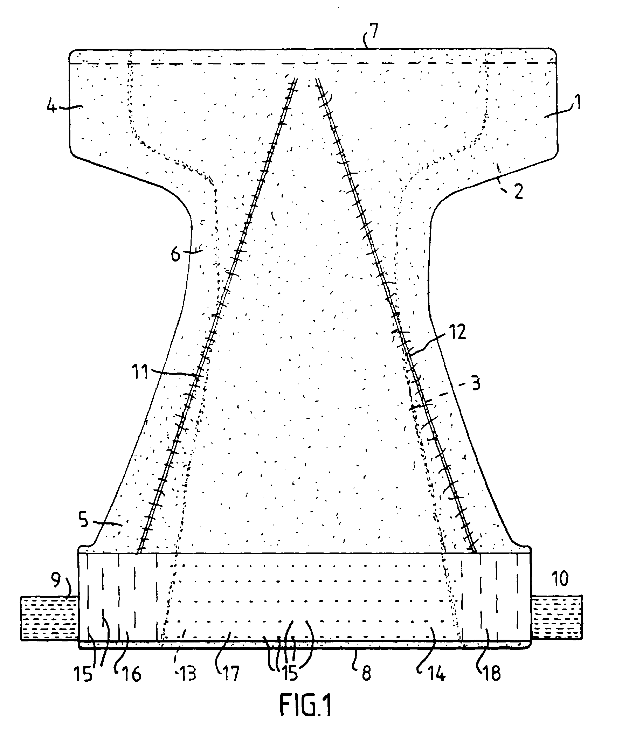

The diaper illustrated in Figure 1 comprises a liquid-permeable casing layer 1, which is intended to face towards the wearer in use, a liquid-impermeable casing layer 2, which is distal from the wearer in use, and an absorbent pad 3 which is enclosed between the casing layers 1, 2. The liquid-permeable casing layer 1 preferably comprises some type of non-woven fabric or perforated plastic film, whereas the liquid-impermeable casing layer 2 may comprise, for instance, a liquid-impermeable plastic film or a hydrophobized non-woven fabric. When worn, the diaper is intended to embrace the lower abdomen of the wearer in a trouser-like fashion, and to this end has a front part 4 which is intended to be placed over the wearer's stomach in use, a rear part 5, which is intended to be placed over the wearer's bottom in use, and a narrower crotch part 6 which is located between the front part 4 and the rear part 5 and which is positioned between the wearer's thighs in use. Along its two short sides, the diaper also presents a front waist edge 7 and a rear waist edge 8, which together form the waist part of the diaper in use. Fastener tabs 9, 10 are disposed along the sides of the diaper rear-part 5, close to the rear waist edge 8. The tabs 9, 10 function to hold the diaper together to form a trouser-like configuration in use, and are therewith fastened to the front part of the diaper against the liquid-impermeable layer. To this end, the front part is preferably provided with a reinforcing layer, within the region in which the tabs shall be fastened. The reinforcing layer may consist, for instance, of a polypropylene plastic strip and functions to enable the tabs to be refastened.

Elastic devices 11, 12, for instance in the form of elastic bands or yarn-spun threads, extend from the centre of the front waist edge 7 of the diaper in a V-shaped pattern towards the rear waist edge 8 of said diaper. A further elastic device 13 in the form of a broad rubber band or a band made of elastic foam material extends along the rear waist edge 8, between the two fastener tabs 9, 10. When the diaper is worn, the elastic devices 11, 12, positioned in a V-shaped pattern, form the elastication around the edges of the diaper legs, whereas the elastic device 13 attached along the rear waist edge 8 forms the elastication around the edge of the waist part of the diaper. This latter elastic device is placed within a casing 14 of heat-meltable fabric surrounding said device, and is secured to the casing at discrete bonding locations 15.

The elastic device 13 is divided into three separate regions 16, 17, 18, having mutually different bonding patterns. A first bonding pattern, in which the bonding locations 15 have the form of discontinuous lines extending transversely to the direction in which the device 13 acts, is disposed in the two regions 16, 18 which lie closest to the fastener tabs. In the region 17 at the centre part of the elastic device 13, the bonds are disposed in punctiform rows instead.

The bonds located in said region 17 cover a smaller area of the elastic device 13 than the bonds located within the regions 16, 18 nearest the fastener tabs 9, 10, and have a small extension transversely to the direction in which the elastic device 13 acts. In this way, the elastic device 13 will retain essentially the same degree of elasticity within this region 17 as in its non-bonded state.

Within the regions 16, 18 located nearest the diaper fastener tabs 9, 10, where the bonds are disposed with small extension in the action direction of the elastic device, but extend substantially perpendicularly to said direction, the bonded elastic device 13 has lost practically all of its elasticity. The greater bonding density within these regions 16, 18 also contributes to reducing the elasticity of the device 13. As before mentioned, the inelastic regions 16, 18 are intended to ensure that the tabs 9, 10 will fasten effectively.

The arrangement of bonding regions in the form of broken lines avoids the occurrence of channels which extend transversely across the full width of the elastic device and through which liquid is able to run when the diaper is worn. Instead, discontinuous folds are formed in the casing 14 around the elastic device 13, these folds preventing the through-passage of liquid but permitting air and water vapour to pass therethrough.

As illustrated in Figure 1, the elastic band 13 with surrounding casing 14 is attached to the liquid-permeable casing layer 1 of the diaper while in a stretched state, for instance with the aid of ultrasonic welding techniques. If the elastic band is manufactured separately, outside the diaper manufacturing line, the actual attachment of the band is effected in the elastic region 16, 18, whereas only a few attachment points are used in the region 17, therewith to reduce the elasticity of the band and to ensure that no space is formed between the band 13 and the casing layer 1 when the band contracts in this region in the finished product. According to one preferred embodiment, the band 13 will have an incomplete bonding pattern when applied to the casing layer 1, and the bonding pattern is completed by fastening the band to said casing layer. Naturally, the band can also be glued firmly to the casing layer, in which case only a few glue points, or preferably longitudinally extending glue beads will occur in the region 17.

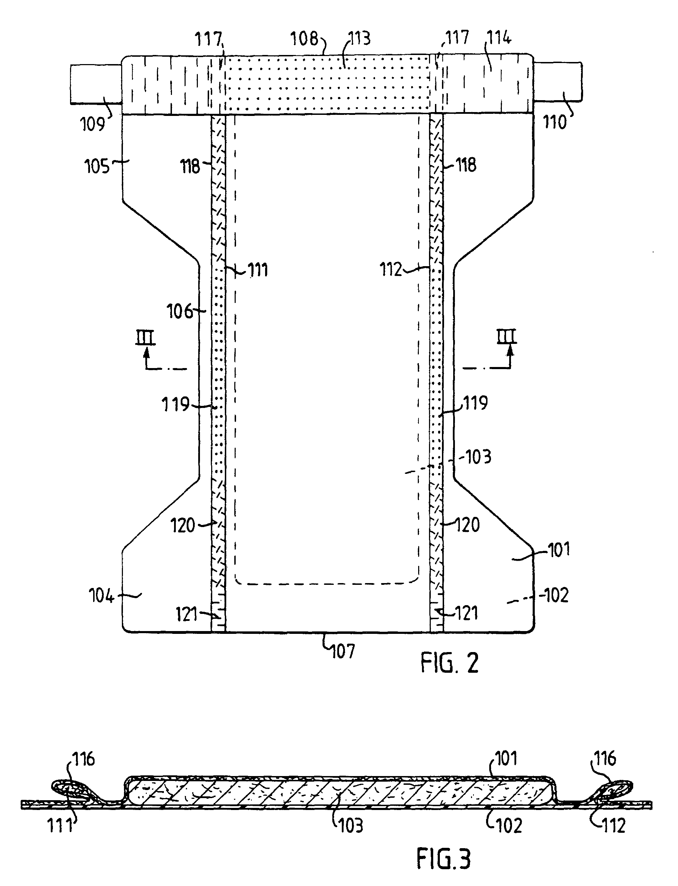

The diaper illustrated in Figure 2 is constructed in essentially the same manner as the diaper illustrated in Figure 1 and includes a liquid-permeable casing layer 101, a liquid-impermeable casing layer 102, and an absorbent pad 103 enclosed between said casing layers.

The diaper has an hour-glass configuration and, similar to the diaper of Figure 1, has a front part 104, a rear part 105, a crotch part 106, and a front and a rear waist edge 107, 108. Diaper fastener tabs 109, 110 are provided on the side edges of the rear part 105, close to the rear waist edge 108. An elastic device 113 is attached along the rear waist edge 108, within a non-woven fibre casing 114, with a bonding pattern corresponding to the diaper illustrated in Figure 1. The diaper illustrated in Figure 2 also includes two further elastic devices 111, 112 of the same kind as the first elastic device, but positioned in the longitudinal direction of the diaper on either side of the absorbent pad 103. These devices 111, 112 are intended to provide the diaper leg elastication. The devices are enclosed in longitudinally extending folds 116 in the liquid-permeable casing layer 101 of the diaper, as will best be seen from Figure 3. Similar to the diaper illustrated in Figure 1, the elastic devices 111, 112 are secured within the folds 116 by ultrasonic welding, wherein the ultrasound perforates the elastic devices 111, 112 in a predetermined pattern and fuses together the surrounding casing parts 114 through the perforations. Each of the elastic devices 111, 112 presents five regions 117-121 having three mutually different bonding patterns and different degress of elasticity. The greatest elasticity is found in the elastic devices 111, 112 within the crotch part 106 of the diaper, where the bonding pattern consists of discrete, punctiform bonds. A bonding pattern in the form of broken, transverse lines has been used within the regions 117, 121 nearest the waist edges 107, 108. As before mentioned, this bonding pattern causes the elastic devices 111, 112 to be practically inelastic within these regions.

The elastic devices 113 are bonded with a pattern of intersecting, broken, oblique lines in those regions 118, 120 located between the inelastic regions 117, 121 at the waist edges 107, 108 of the diaper and the regions 119 in the crotch part 106 thereof. This bonding pattern results in a lower elasticity than the punctiform bonds at the crotch part of the diaper, but in greater elasticity than the transverse bonds at the waist edges of the diaper.

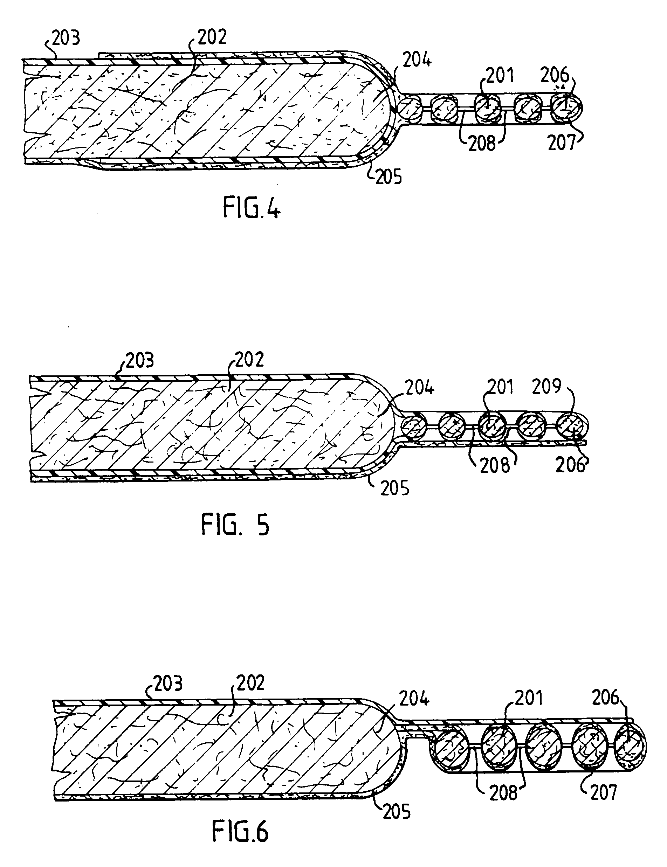

Figures 4-8 illustrates examples of methods of securing an inventive elastic device to one edge of a diaper or to some other absorbent product. Corresponding elements in the Figures have been identified with the same reference signs. In all of the embodiments illustrated in Figures 4-8, the elastic device consists of an elastic strip of open-cell foamed plastic.

The elastic device 201 illustrated in Figure 4 is attached in the extension of an absorbent pad 202, which has a liquid-impermeable layer 203 attached to one side thereof, i.e. the outwardly facing side thereof. The liquid-impermeable layer is folded around the edge part 204 of the absorbent pad, so as to prevent the leakage of fluid passed said edge. A liquid-permeable layer 205, for instance a polypropylene non-woven fabric layer, is attached to the other side, the inwardly facing side, of the absorbent pad 202. The liquid-permeable layer 205 extends beyond the absorbent pad 202 and over the elastic device 201, and is folded back around the free edge 206 of said device and fastened, e.g. with the aid of melt adhesive, to the liquid-impermeable layer 203 on the outer side of the absorbent pad 202. The elastic device 201 is thus enclosed in a fold 207 located externally of the absorbent pad 202 in the liquid-permeable layer 205 and is secured within the fold 207 at discrete bonding locations 208, in accordance with the invention.

In the embodiment illustrated in Figure 5, the elastic device 201 is secured within a fold 209 in the liquid-impermeable layer 203, located externally of the edge 204 of the absorbent pad. This layer 203 normally comprises plastic film and in order to avoid contact between the plastic film and the wearer's skin in use, the liquid-permeable layer 205 extends on the inside of the diaper completely past the edge 204 of the absorbent pad, to the free edge 206 of the elastic device.

Figure 6 illustrates a further example of a method of securing the elastic device 201 within a fold 207 in the liquid-permeable layer 205. The liquid-impermeable layer 205 extends beyond the absorbent pad 202 and is folded around the elastic device 201, without being folded back across the pad 202. The liquid-impermeable layer 203 extends on the outside of the pad 202 up to the free edge 206 of the elastic device. An extremely effective leak-proof construction is achieved in this way.

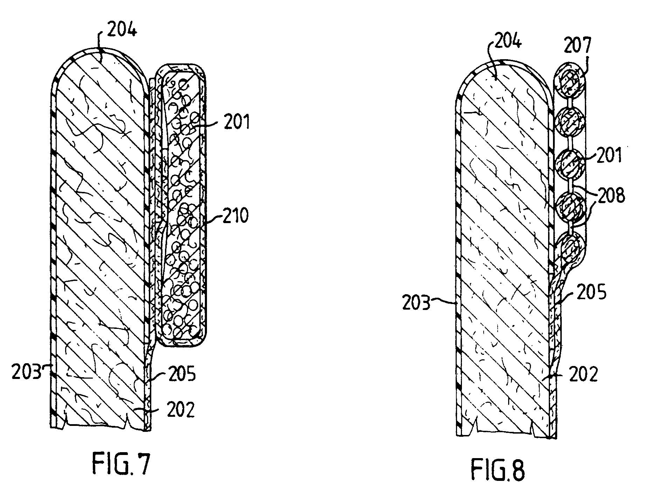

The elastic device 201 illustrated in Figure 7 is secured within a casing 210 of heat-meltable non-woven fabric. The device 201 with surrounding casing 210 is attached to the liquid-permeable layer 205 on the inside of the pad 207 and inwardly of the edge 204 of said pad.

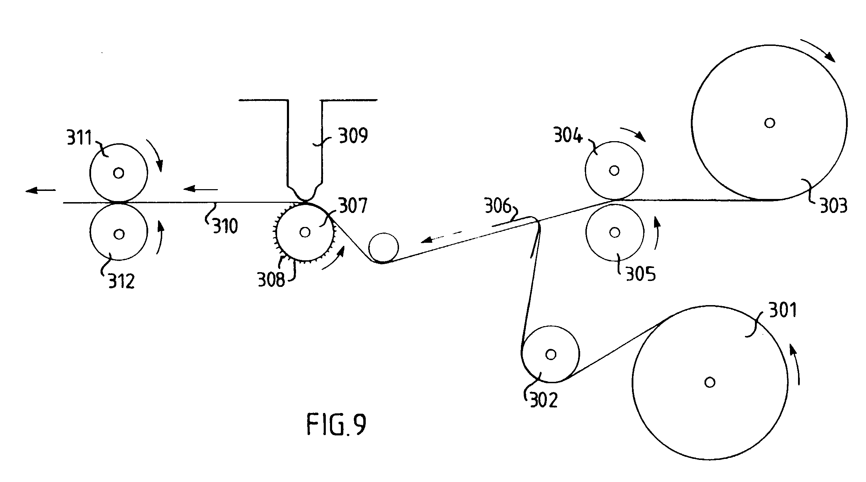

The elastic device 201 illustrated in Figure 8 is also secured inwardly of the edge 204 of the absorbent pad. In this embodiment, however, the device 201 is secured within a fold 207 in the liquid-permeable layer 205. The liquid-impermeable layer 203 in Figures 7 and 8 is folded around the edge 204 of the absorbent pad in the same manner as in the Figure 4 embodiment.

An inventive elastic device can be secured within a casing in the manner illustrated schematically in Figure 9. A heat meltable casing material 301, for instance in the form of non-woven fabric or plastic film, extends from a first reel of material, via a guide roller 302. At the same time, a band 303 of elastic material is fed from a second reel while passing between a first pair of rollers 304, 305, whereafter the casing material 301 is folded around the elastic band 304 by means of a folding plate 306, such that both sides of the band 303 are covered by the casing material 301. The band 303, together with the surrounding casing 301, is then transported over a patterned bonding roller 307. The bonding roller 307 presents raised portions or devices 308 which correspond to the desired bonding pattern of the finished elastic laminate. Bonding is effected with the aid of an ultrasonic horn 309. The ultrasound perforates the elastic band and fuses the casing material together through the perforations thus formed. The bonding pattern is predetermined and, as before mentioned, is controlled by the raised portions or devices 308 on the bonding roller. The resultant, bonded elastic laminate 310 is then advanced by a second pair of rollers 311 and 312 which are driven at a second speed which is higher than the speed at which the first roller pair 304, 305 is driven. Because the second roller pair 311, 312 is driven at a higher speed than the first roller pair 304, 305, the elastic band 303 will be stretched before it is bonded within the heat-meltable casing 301.

The elasticity of the finished laminate 310 can be controlled in two ways. By selecting a given ratio between the speeds at which the first roller pair 304, 305 and the second roller pair 311, 312 are driven, it is possible to impart to the elastic laminate 310 a given maximum elasticity or basic elasticity. By using different bonding patterns for different parts of the elastic band, it is possible to reduce to varying high degrees the basic elasticity determined by the speeds at which said roller pairs are driven. The finished elastic laminate 310 will thus present parts of mutually different elasticity.

In order to illustrate how elasticity is affected by the bonding pattern, tests were carried out on two laminate samples having mutually different bonding patterns. The elastic devices used in both samples had a width of 50 mm and a thickness of 2 mm and comprised a band of flexible, polyurethane foam based on polyester. The elastic foam material is retailed by CIRRUS A/S, Denmark, under the designation 2 130 170. The elastic band was enclosed in a non-woven fabric casing or envelope in the manner described with reference to Figure 9. The band was therewith stretched to 70%, meaning that the ratio between the first driving speed and the second driving speed was 1.70. The non-woven fabric casing used comprise heat-bonded polypropylene fibres.

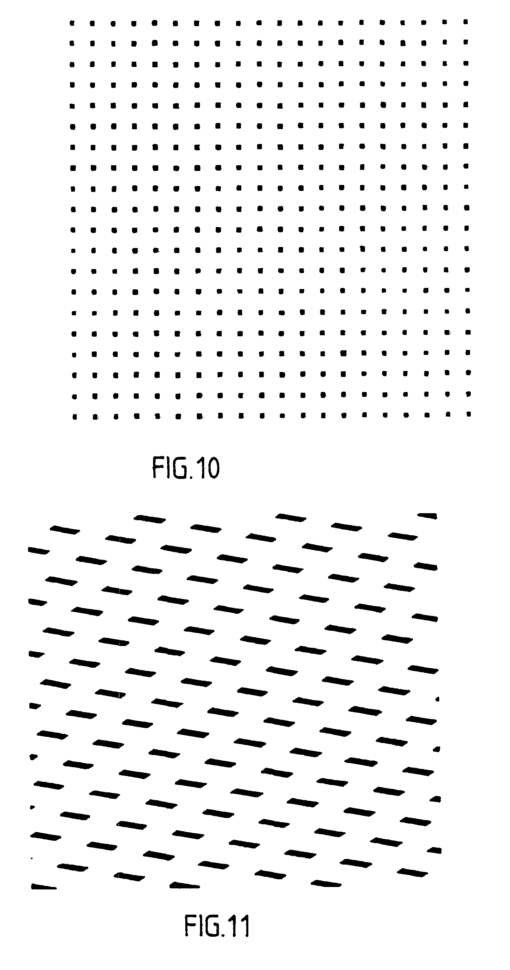

The first bonding pattern a) was in the form of small, discrete square bonding locations, as illustrated in Figure 10, whereas the second bonding pattern b), illustrated in Figure 11, consisted of broken lines which extended substantially in the cross-direction of the elastic band.

Both samples a) and b) were then subjected to tensile tests on an INSTRON 1122, in order to compare their elasticities.

Each sample was tested in the following manner:
A sample having a length of 200 mm was secured firmly between the jaws of the INSTRON 1122. The sample was stretched to a load of 10 N. The jaws were then moved towards one another until the tensile stress in the sample was 0 N. The sample was then allowed to rest for two minutes, in order to eliminate any residual stretch. The tensile stress was then again adjusted to 0 N.

The samples were stretched three times between the 0-level determined in the aforesaid manner and a tensile stress of 10 N. The jaws were moved together at a speed of 300 mm/min. and the sample contracting force when relaxing the tension was recorded as a function of contraction in mm. The results obtained with these samples are shown in diagram 1, in which each curve represents an average value of the three stretch tests carried out. The sample a) contracted through 75 mm, whereas the sample b) only contracted through 40 mm. Thus, a bonding pattern in which the bonding locations extend substantially perpendicularly to the direction in which the elastic device acts greatly reduces the elasticity of said device, whereas a punctiform bonding pattern will only slightly influence the elasticity of said device.

The total surface area of the bonds is also significant to the elasticity of the device, in addition to the configuration of the bonds and their orientation in relation to the direction in which the elastic device stretches or acts. The larger the area on the elastic device that is taken-up by the bonding locations, the smaller the degree of elasticity of said device.

Although the inventive elastic device has been described with reference to diapers, it will be understood that the device can be used in a number of other applications. For instance, the invention can be applied in the manufacture of surgical dressings. protective clothing, underwear and sport-sware.

In the Figures of the accompanying drawings, the inventive elastic device has been shown as a single elastic band. It will be understood, however, that the elastic device may consist of two or more separate bands.

The inventive elastic device can also be used to particular advantage in diapers having double leg elastication, i.e. such diapers as those in which each leg opening has an inner and an outer elastic edge. For instance, in this case, either the inner or the outer elastic edge is formed from an inventive elastic device, whereas the other elastic edge is of a conventional kind. Naturally, both edges can be provided with inventive elastic devices.

It is also conceivable to secure an inventive I elastic device on the inside of a diaper, supplemented with a corresponding device of the same or a different kind on the outside of the diaper. This additional elastic device will prevent the diaper from ballooning from the wearer's body in use.

Other modifications are conceivable within the scope of the following Claims.

## Claims

1. A method of securing an elastic band (13) between two material layers (14) which at least partially consist of meltable material, wherein perforations in the form of holes and/or slots are formed in the elastic band (13): in that the band is placed between the two material layers; the material layers opposite said perforations are mutually bonded by melt fusion through said perforations so that the elastic band is held mechanically between the material layers; the perforations are formed in the elastic band in the same operation as the material layers are fused together; and the elastic band (13) is placed between the two material layers (14) while in a stretched state **characterised in that** the elastic band consists of one or more separate bands of elastic foam material having open or closed cells.

2. A method according to Claim 1, **characterized in that** the elastic band is pretensioned by being stretched uniformly, is positioned between the material layers (301) and is caused to pass over a bonding roller (308) which has a pattern of raised portions thereon and which functions to bond the elastic band (304) to the material layers (301) surrounding said band, with the aid of ultrasound (309), wherein the ultrasound perforates the elastic device and fuses together the two material layers through the perforations in a bonding pattern corresponding to the pattern on the bonding roller.

3. An elastic band (13) secured between two material layers (14) for use in articles which are intended for one-time use only, such as disposable diapers, sanitary napkins, surgical dressings, protective clothing or the like, wherein the material layers (14) at least partially consist of meltable material, wherein the elastic band (13) is secured in a stretched state between the two material layers (14) and presents perforations in the form of holes and/or slots through which the material layers located on both sides of the band (13) are mutually joined together by melt fusion in a punctiform and/or linear bonding pattern so that the elastic band is held mechabically between the material layers, **characterised in that** the elastic band consists of one or more separate bands of elastic foam material having open or closed cells.

4. An elastic band (13) according to Claim 3, **characterized in that** the band presents at least two regions (16, 18) of mutually different bonding patterns and of mutually different elasticity in the direction in which the band acts.

5. An elastic band according to Claim 3 or 4, **characterized in that** it presents at least one region (17) of substantially punctiform perforations through which the two material layers are bonded together.

6. An elastic band acording to Claim 3, 4 or 5, **characterized in that** it presents at least one region (16, 18) with perforations which have a smaller extension in the direction in which the device acts than perpendicularly to said direction and through which the two material layers (14) are bonded together.

7. An elastic band according to any one of Claims 3-6, **characterized in that** the foam material is a polyester-based polyurethane foam.

8. A diaper comprising a liquid-permeable casing layer (1), which is intended to face the wearer in use, a liquid-impermeable casing layer (2), which is intended to lie remote from the wearer in use, and an absorbent pad (3) located between said two layers (1, 2), and which diaper has a front part (4) which is intended to be located forwardly on the wearer in use, a rear part (5) which is intended to be located rearwardly on the wearer in use, and a crotch part (6) which is located between the front part (4) and the rear part (5) and which is intended to be placed between the thighs of the wearer in use, so that the diaper, when worn, embraces the lower abdomen of the wearer in a trouser-like fashion and therewith presents a waist line (7, 8) around the waist of the wearer and a leg line around each of the user's thighs, the diaper further including at least one elastic band (13) which is secured in a stretched state between two material layers (14) and which presents perforations in the form of holes and/or slots (15), wherein the material layers (14) located on opposite sides of the band are at least partially comprised of a meltable material and are mutually bonded by melt fusion in a punctiform and/or linear bonding pattern through said holes and/or slots (15), so that the elastic band (13) is held mechanically between the material layers (14); and the elastic band (13) is affixed along the whole of at least one of the diaper lines or at least along a part of at least one of the diaper lines and at least one of the material layers (14) is joined to one of the casing layers in at least one of the diaper line parts, **characterised in that** the elastic band consists of one or more separate bands of elastic foam material having open or closed cells.

9. A diaper according to Claim 8, **characterized in that** at least one of the material layers (207, 209, 210) surrounding the elastic band (201) comprises one of the diaper casing layers.

10. A diaper according to Claim 9, **characterized in that** the liquid-permeable casing layer (205) is folded over the elastic band (201).

11. A diaper according to Claim 9, **characterized in that** the liquid-impermeable casing layer (203) is folded over the elastic band (201).

12. A diaper according to Claim 8, **characterized in that** the elastic band (201) is encased in a separate casing (210) of meltable material.

13. A diaper according to any one of Claims 8-12, **characterized in that** an elastic band (13) is attached at least along the waist line on the rear part (5) of the diaper; **in that** the diaper has fastener tabs (9, 10) which function to secure the diaper in a trouser-like configuration, said fastener tabs (9, 10) being affixed to the sides of the rear part (5) of the diaper in the close proximity of the waist line (7, 8); and **in that** the elastic band (13) presents a pattern of perforations (15) which extend substantially transversely across the band within those regions (16, 18) of the waist line (7, 8) at which the fastener tabs (9, 10) are affixed.

14. A diaper according to any one of Claims 8-13, **characterized in that** an elastic band (111, 112) is affixed to each leg line; and **in that** said bands each present at least two regions (116-120) of mutually different bonding patterns and therewith mutually different degrees of elasticity.

15. A diaper according to Claim 14, **characterized in that** each of the elastic bands (111, 112) presents at least three regions (117-121) of mutually different bonding patterns and therewith mutually different degrees of elasticity, of which regions the region (119) located in the crotch part (106) of the diaper has the greatest elasticity.

16. A diaper according to any one of Claims 8-13, **characterized in that** it includes further elastic devices (11, 12) such as elastic bands or threads which are affixed to the diaper by gluing or welding.

## Patentansprüche

1. Verfahren zum Befestigen eines elastischen Bandes (13) zwischen zwei Materialschichten (14), die wenigstens teilweise aus schmelzbarem Material bestehen, worin im elastischen Band (13) Perforationen in Form von Löchern und/oder Schlitzen gebildet werden, das Band zwischen die beiden Materialschichten gelegt wird, die Materialschichten gegenüber den Perforationen durch diese Perforationen hindurch mit Hilfe einer Schmelzschweissung miteinander verbunden werden, so dass das elastische Band zwischen den Materialschichten mechanisch gehalten wird, die Perforationen in elastischen Band im selben Arbeitsgang hergestellt werden, in dem die Materialschichten miteinander verschweisst werden, und das elastische Band (13) zwischen den beiden Materialschichten (14) angeordnet wird, während es sich in einem gestreckten Zustand befindet, **dadurch gekennzeichnet, dass** das elastische Band aus einem oder mehreren, getrennten Bändern aus elastischem Schaummaterial besteht, das offene oder geschlossene Zellen aufweist.

2. Verfahren nach Anspruch 1, **dadurch gekennzeichnet, dass** das elastische Band gleichmässig vorgespannt wird, dass es zwischen die Materialschichten (301) gelegt wird und dass es dazu gebracht wird, über einer Verbindungswalze (207) vorbeizulaufen, auf der sich ein Muster von erhabenen Teilen befindet, und die dazu dient, das elastische Band (303) mit Hilfe von Ultraschall (309) mit den Materialschichten (301) zu verbinden, die das Band umgeben, wobei der Ultraschall das elastische Teil perforiert und die beiden Materialschichten durch diese Perforationen hindurch in einem Verbindungsmuster zusammenschmilzt, das dem Muster auf der Verbindungswalze entspricht.

3. Elastisches Band (13), das zur Verwendung bei Artikeln, die für einmaligen Gebrauch bestimmt sind, beispielsweise bei Windeln, Damenbinden, chirurgische Bekleidungsstücken, Schutzkleidung oder dergleichen, zwischen zwei Materialschichten befestigt ist, wobei die Materialschichten (14) wenigstens teilweise aus schmelzbaren Material bestehen, wobei das Band (13) in gestrecktem Zustand zwischen den Materialschichten (14) gesichert ist, und Perforationen in Form von Löchern und/oder Schlitzen hat, durch die hindurch die Materialschichten, die auf beiden Seiten des Bandes (13) liegen, durch eine Verschmelzung in Punktform und/oder einem linearen Verbindungsmuster miteinander verbunden werden, **dadurch gekennzeichnet, dass** das elastische Band aus einem oder mehreren, getrennten Bändern aus elastischem Schaummaterial besteht, das offene oder geschlossene Zellen aufweist.

4. Elastisches Band (13) nach Anspruch 3, **dadurch gekennzeichnet, dass** das Band wenigstens zwei Bereiche (16, 18) mit unterschiedlichen Verbindungsmustern und von gegenseitig unterschiedlicher Elastizität in der Richtung aufweist, in der das Band wirkt.

5. Elastisches Band nach einem der Ansprüche 3 oder 4, **dadurch gekennzeichnet, dass** es wenigstens einen Bereich (17) mit im Wesentlichen punktförmigen Perforationen aufweist, durch die hindurch die beiden Materialschichten miteinander verbunden sind.

6. Elastisches Band nach einem der Ansprüche 3, 4 oder 5, **dadurch gekennzeichnet, dass** es wenigstens einen Bereich (16, 18) mit Perforationen hat, die in der Richtung, in der das Band wirkt, eine kleinere Ausdehnung haben als senkrecht zu dieser Richtung, wobei durch diese Perforationen hindurch die beiden Materialschichten (14) miteinander verbunden sind.

7. Elastisches Band nach einem der Ansprüche 3 - 6, **dadurch gekennzeichnet, dass** das Schaummaterial ein Polyurethanschaum auf Polyesterbasis ist.

8. Windel mit einer flüssigkeitsdurchlässigen Deckschicht (1), die dazu bestimmt ist, beim Gebrauch auf die Trägerperson zuzuweisen, einer flüssigkeitsundurchlässigen Deckschicht (2), die dazu bestimmt ist, im Gebrauch von der Trägerperson wegzuweisen und einem absorbierenden Kissen (3), das zwischen den beiden Schichten (1 und 2) liegt, wobei diese Windel einen Vorderteil (4) hat, der dazu bestimmt ist, beim Gebrauch vorne an der Trägerperson anzuliegen, einen hinteren Teil (5), der dazu bestimmt ist, beim Gebrauch hinter der Trägerperson zu liegen, sowie einen Schritteil (6), der zwischen dem Vorderteil (4) und dem hinteren Teil (5) liegt und der dazu bestimmt ist, beim Gebrauch zwischen den Schenkeln der Trägerperson zu liegen, so dass die Windel beim Tragen den Unterleib der Trägerperson in hosenähnlicher Form umgibt und damit eine Gürtellinie (7, 8) um den Bauch der Trägerperson sowie eine Beinlinie um jeden den Schenkel der Trägerperson aufweist, wobei die Windel ferner wenigstens ein elastisches Band (13) aufweist, das in gestrecktem Zustand zwischen den beiden Materialschichten (14) befestigt ist und das Perforationen in Form von Löchern und/oder Schlitzen (15) aufweist, worin die Materialschichten (14), die an einander gegenüberliegenden Seiten des Bandes angeordnet sind, wenigstens teilweise aus schmelzbarem Material bestehen und dass sie durch einen Schmelzvorgang in einem punktförmigen und/oder linienförmigen Verbindungsmuster durch diese Löcher und/oder Schlitze (15) aneinander befestigt werden, so dass das elastische Band (13) mechanisch zwischen den Materialschichten (14) festgehalten wird; und das elastische Band (13) längs der gesamten Länge wenigstens einer der genannten Windellinien oder längs wenigstens eines Teiles wenigstens einer dieser Windellinien und an wenigstens einer Materialschicht (14) befestigt wird, die mit einer der Deckschichten in wenigstens einer der Windellinienteile verbunden ist, **dadurch gekennzeichnet, dass** das elastische Band aus einem oder mehreren, getrennten Bändern aus elastischem Schaummaterial besteht, das offene oder geschlossene Zellen aufweist.

9. Windel nach Anspruch 8, **dadurch gekennzeichnet, dass** wenigstens eine Materialschicht (207, 209, 210), die das elastische Band (201) umgibt, eine der Windeldeckschichten umfasst.

10. Windel nach Anspruch 9, **dadurch gekennzeichnet, dass** die flüssigkeitsdurchlässige Deckschicht (205) über das elastische Band (201) gefaltet ist.

11. Windel nach Anspruch 9, **dadurch gekennzeichnet, dass** die flüssigkeitsundurchlässige Deckschicht (203) über das elastische Band (201) gefaltet ist.

12. Windel nach Anspruch 8, **dadurch gekennzeichnet, dass** das elastische Band (201) in einer getrennten Hülle (210) aus schmelzbarem Material eingeschlossen ist.

13. Windel nach einem der Ansprüche 9 bis 12, **dadurch gekennzeichnet, dass** das elastische Band (13) wenigstens längs der Gürtellinie am hinteren Teil (5) der Windel angebracht ist; dass die Windel Befestigungslappen (9, 10) hat, die dazu dienen, die Windel in hosenähnlicher Form festzuhalten, wobei die Befestigungslappen (9, 10) an den Seiten des hinteren Teiles (5) der Windel in enger Nachbarschaft mit der Gürtellinie (7, 8) angebracht sind; und dass das elastische Band (13) ein Muster von Perforationen (15) hat, die innerhalb derjenigen Bereiche (16, 18) der Gürtellinie (7, 8), an denen die Befestigungslappen (9, 10) befestigt sind, im Wesentlichen quer über das Band verlaufen.

14. Windel nach einem der Ansprüche 8 bis 13, **dadurch gekennzeichnet, dass** das elastische Band (111, 112) an jeder Beinlinie befestigt ist und dass diese Bänder jeweils wenigstens zwei Bereiche (116 bis 120) gegenseitig unterschiedlicher Verbindungsmuster und damit gegenseitig unterschiedlicher Elastizitätsgrade aufweisen.

15. Windel nach Anspruch 14, **dadurch gekennzeichnet, dass** jedes elastische Band (111, 112) wenigstens drei Bereiche (117 bis 121) gegenseitig unterschiedlicher Verbindungsmuster und damit gegenseitig unterschiedlicher Grade von Elastizität aufweisen, wobei derjenige Bereich (119), der im Schrittbereich (106) der Windel liegt, die größte Elastizität hat.

16. Windel nach einem der Ansprüche 8 bis 13, **dadurch gekennzeichnet, dass** sie weitere elastische Teile (11, 12) aufweist, beispielsweise elastischer Bänder oder Fäden, die durch eine Kleb- oder Schweissvorgang an der Windel befestigt sind.

## Revendications

1. Procédé de fixation d'une bande élastique (13) entre deux couches de matière (14) qui sont faites au moins partiellement d'un matériau fusible, dans lequel des perforations ayant la forme de trous et/ou de fentes sont formées dans la bande élastique (13); la bande est placée entre les deux couches de matière; les couches de matière en face desdites perforations sont liées l'une à l'autre par thermofusion à travers lesdites perforations, de sorte que la bande élastique est maintenue mécaniquement entre les deux couches de matière; les perforations sont formées dans la bande élastique au cours de la même opération que celle au cours de laquelle les couches de matière sont fusionnées; et la bande élastique (13) est placée entre les deux couches de matière (14) alors qu'elle se trouve à l'état étiré; **caracterisé en ce que** la bande élastique comprend une ou plusieurs bandes séparées, faites d'un matériau de mousse élastique comportant des cellules ouvertes ou fermées.

2. Procédé selon la revendication 1, **caracterisé en ce que** la bande élastique est mise en précontrainte par un étirage uniforme; est placée entre les couches de matière (301) et est amenée à passer sur un rouleau de liaison (308), qui présente un motif de parties en relief et qui sert à lier, avec l'aide d'un appareil à ultrasons (309), la bande élastique (304) aux couches de matière (301) qui entourent cette bande; sachant que les ultrasons perforent la bande élastique et font fusionner les deux couches de matière à travers les perforations suivant un motif de liaison qui correspond au motif porté par le rouleau de liaison.

3. Bande élastique (13) fixée entre deux couches de matière (14), à utiliser dans les articles destinés à un usage unique tels que des couches-culottes, des serviettes hygiéniques, des vêtements chirurgicaux, des vêtements de protection jetables, ou autre produits similaires; sachant que les couches de matière (14) sont faites au moins partiellement d'un matériau fusible; et sachant que la bande élastique (13) est fixée à l'état étiré entre les deux couches de matière (14) et qu'elle présente des perforations sous forme de trous et/ou de fentes à travers lesquelles les couches de matière situées des deux cotés de la bande (13) sont réunies l'une à l'autre par thermofusion suivant un motif de liaison punctiforme et/ou linéaire, de sorte que la bande élastique (13) est maintenue mécaniquement entre les deux couches de matière (14); **caracterisée en ce que** la bande élastique comprend une ou plusieurs bandes séparées, faites d'un matériau de mousse élastique comportant des cellules ouvertes ou fermées.

4. Bande élastique (13) selon la revendication 3, **caracterisée en ce que** la bande présente au moins deux régions (16,18) avec des motifs de liaison différents l'un de l'autre et des élasticités différentes l'une de l'autre dans la direction dans laquelle agit la bande.

5. Bande élastique selon la revendication 3 ou 4, **caracterisée en ce que** la bande présente au moins une région (17) à perforations sensiblement punctiformes à travers lesquelles les deux couches de matière sont liés ensemble.

6. Bande élastique selon la revendication 3, 4 ou 5, **caracterisée en ce que** la bande présente au moins une région (16,18) avec des perforations qui ont une plus petite longueur dans la direction dans laquelle la bande agit que perpendiculairement à cette direction et à travers lesquelles les deux couches de matière (14) sont liées ensemble.

7. Bande élastique selon l'une quelquonque des revendications 3 à 6, **caracterisée en ce que** le matériau de mousse est une mousse de polyuréthane à base de polyester.

8. Couche-culotte qui comprend une couche d'enveloppe (1) perméable aux liquides, destinée à être en vis-à-vis de l'utilisateur en cours d'utilisation, une couche d'enveloppe (2) imperméable aux liquides, destinée à se trouver loin de l'utilisateur en cours d'utilisation, et un coussin absorbant (3) placé entre lesdites deux couches (1,2); couche-culotte qui comporte une partie avant (4) destinée à se trouver placée devant l'utilisateur en cours d'utilisation, une partie arrière (5) destinée à se trouver placée derrière l'utilisateur en cours d'utilisation, et une partie d'entrejambe (6) placée entre la partie avant (4) et la partie arrière (5) et destinée à se trouver placée entre les cuisses de l'utilisateur en cours d'utilisation, de telle sorte que la couche-culotte, quand elle est portée, entoure le bas de l'abdomen de l'utilisateur à la façon d'une culotte et comporte une ligne de taille (7,8) autour de la taille de l'utilisateur et une ligne de jambe autour de chaque cuisse de l'utilisateur, la couche-culotte comportant en outre au moins une bande élastique (13) qui est fixée à l'état étiré entre deux couches de matière (14) et qui présente des perforations en forme de trous et/ou de fentes (15), sachant que les couches de matière (14) placées sur les côtés opposés de la bande sont au moins partiellement faites d'un matériau fusible et sont liées l'une à l'autre par thermofusion suivant un motif de liaison punctiforme et/ou linéaire à travers lesdits trous et/ou fentes (15), de sorte que la bande élastique (13) est maintenue mécaniquement entre les couches de matière (14), et sachant que la bande élastique (13) est fixée sur toute la longueur d'au moins une des lignes de la couche-culotte ou tout au moins sur une partie de l'une au moins des lignes de la couche-culotte, et sachant que l'une au moins des couches de matière (14) est réunie à l'une des couches d'enveloppe dans l'une au moins des parties des lignes de la couche-culotte, **caracterisée en ce que** la bande élastique comprend une ou plusieurs bandes séparées, faites d'un matériau de mousse élastique comportant des cellules ouvertes ou fermées.

9. Couche-culotte selon la revendication 8, **caracterisée en ce que** l'une au moins des couches de matière (207,209,210) qui entourent la bande élastique (201) est l'une des couches d'enveloppe de la couche-culotte.

10. Couche-culotte selon la revendication 9, **caracterisée en ce que** la couche d'enveloppe perméable aux liquides (205) est repliée sur la bande élastique (201).

11. Couche-culotte selon la revendication 9, **caracterisée en ce que** la couche d'enveloppe imperméable aux liquides (203) est repliée sur la bande élastique (201).

12. Couche-culotte selon la revendication 8, **caracterisée en ce que** la bande élastique (201) est enfermée dans une enveloppe séparée (210) de matière fusible.

13. Couche-culotte selon l'une quelquonque des revendications 8 à 12, **caracterisée en ce qu'**une bande élastique (13) est fixée au moins le long de la ligne de taille, sur la partie arrière (5) de la couche-culotte, **en ce que** la couche-culotte comporte des pattes d'attache (9,10) qui servent à immobiliser la couche-culotte dans une configuration de culotte, lesdites pattes d'attache (9,10) étant fixées aux côtés de la partie arrière (5) de la couche-culotte, au proche voisinage de la ligne de taille (7,8), et **en ce que** la bande élastique (13) présente un motif de perforations (15) qui s'étend sensiblement transversalement sur la bande dans les régions (16,18) de la ligne de taille (7,8) à laquelle sont fixées les pattes d'attache (9,10).

14. Couche-culotte selon l'une quelquonque des revendications 8 à 13, **caractérisée en ce qu'**une bande élastique (111,112) est fixée à chaque ligne de jambe et **en ce que** lesdites bandes présentent chacune au moins deux régions (116-120) avec des motifs de liaison différents les uns des autres et avec des degrés d'élasticité différents les uns des autres.

15. Couche-culotte selon la revendication 14, **caracterisée en ce que** chaque bande élastique (111,112) présente au moins trois régions (117-121) avec des motifs de liaison différents les uns des autres et avec des degrés d'élasticité différents les uns des autres, la région (119) placée dans la partie d'entrejambe (106) de la couche-culotte ayant l'élasticité la plus importante.

16. Couche-culotte selon l'une quelquonque des revendications 8 à 13, **caractérisée en ce qu'**elle comprend en outre des dispositifs élastiques (11,12) tels que des bandes ou des fils élastiques qui sont fixés a la couche-culotte par collage ou soudage.
